# EUROPEAN PATENT SPECIFICATION

(11) **EP 1 394 544 B1**
(45) Date of publication and mention of the grant of the patent: **04.02.2009**
(21) Application number: 03007023.9
(22) Date of filing: 27.03.2003
(51) Int. Cl.: G01N 33/26, C12Q 1/68, C09D 11/00

(54) **Method for mixing ribonulceic acid in water insoluble media and application thereof**
Verfahren zum Mischen von Nukleinsäuren in wasserunlöslichen Medien, und dessen Verwendung
Mèthode de mélange d'acides nucléiques dans un milieu insoluble dans l'eau, et son utilisation

(30) Priority: 30.08.2002 JP 2002294229
(43) Date of publication of application: 03.03.2004
(73) Proprietor: APDN (B.V.I.) Inc., Road Town Tortola (VG)
(72) Inventor: Sheu, Jung-Jei, Chungho City, Taipei County, Taiwan 235 (JP); Liang, Ming-Hwa, Chungho City, Taipei County, Taiwan 235 (JP); Chen, Chung-Shung, Chungho City, Taipei County, Taiwan 235 (JP)
(74) Representative: Kador & Partner

(56) References cited:
- WO-A-02/12560
- WO-A-02/22866
- WO-A-98/06084
- WO-A-98/10649
- WO-A-02/090581

## Description

The invention relates to a method for mixing nucleic acid in water insoluble media by mixing water-soluble nucleic acid with water insoluble media through utilizing a particular intermediate solution, and such media having nucleic acid can be applied on products as anti-counterfeiting labels.

As biotechnology develops, biology-related technologies have been applied not only in the medical and researches related fields, but also on daily-life matters, such as security systems utilizing fingerprints or irises for identification, household appliances controlled by human voice, etc. Since e DNA sequences can be unique and complex, utilization of such particular characteristics in daily-life products or solving problems, such as preventing products from being counterfeited, has become an issue of great interest.

Many products not only utilize apparent and unique designs and qualities to earn consumers' trust, but also add labels for anti-counterfeit purpose. Traditional anti-counterfeiting labels are mostly made of physical or chemical materials, for example, magnetic strips on checkbooks, laser holographs on credit cards, fluorescent ink on stocks, and heat-sensitive inks on confidential documents. Other anti-counterfeiting labels are made by adding antigen to objects and detected with antibody; however, since antigens and antibodies are proteins that characterize inferior stability, especially at high temperature those proteins thereof might lose activity and be destroyed, thus reducing the stability and identification accuracy. In addition, since the reactions of antigens and antibodies provide few variations, counterfeiting can be easily done.

It is widely known by persons skilled in the art of molecule biotechnology that DNA is highly water-soluble, and no attempts have been shown to mix DNA in other water insoluble solvents or media. The known technology enables DNA to be dissolved in water-soluble solutions, and then apply such solutions onto the surface of products as anti-counterfeiting labels. Yet the most critical drawback thereof lies in the fact that water-soluble solutions are not able to firmly attach to material like resin, pottery or glass. Therefore, water-soluble anti-counterfeiting labels applied thereon can easily be destroyed or detached, thus the identification function intended is not to be realized.

For overcoming the foregoing drawbacks of the conventional arts, the invention provides a method for mixing nucleic acid in water insoluble media and the application thereof.

The invention provides a method for mixing nucleic acid in water insoluble media, comprising procedures as follows: respectively dissolving nucleic acid in water-soluble solutions and water insoluble media in solvents, and then mix nucleic acid solution in the media by utilizing a particular intermediate solution, thus media having nucleic acid are obtained.

The foregoing media are polymers, such as polypropylene (PP), polycarbonate (PC) or polystyrene (PS).

The foregoing intermediate solution is an organic solvent, such as ethanol, acetone, chloroform or the mixtures thereof.

Another objective of the invention is to provide a manufacturing method of anti-counterfeiting labels for products, with characteristics being that: mixing known sequences nucleic acid in water insoluble media by using the method described above, and then labeling the media on the surface of objects or enabling the media to infiltrate in or mix with such objects as labels. The foregoing media are utilized for mixing and protecting nucleic acid, and for adhering or mixing in other objects.

The foregoing objects can be liquids or solid objects, wherein liquid objects, such as inks, paints, pigments, cosmetics, seals or glues, can be mixed with media having ribonucleic acid, thus enabling such liquids to be anti-counterfeiting labels. In addition, solid objects, such as antiques, jewelries, credit cards, magnetic strip cards, souvenirs, can be attached thereon with media having nucleic acid, thus enabling such solid objects to provide anti-counterfeiting functions. Apart from that, media having nucleic acid can be utilized directly as material for products, thus such products are to contain anti-counterfeiting functions.

The nucleic acid is a general term for deoxyribonucleic acid (DNA) or ribonucleic acid (RNA), and can be chosen from animals, plants, bacteria, viruses, fungi, or synthetic vectors or fragments et al.

The authentication method by utilizing nucleic acid as product anti-counterfeiting labels mentioned above is to detach the media from objects, and then extract nucleic acid in such media by utilizing solvents, eventually, being amplified through the PCR reaction, the authenticity of objects can be verified.

These and other features, aspects and advantages of the present invention will become better understood with regard to the following description, appended claims and accompanying drawings that are provided only for further elaboration without limiting or restricting the present invention, where:

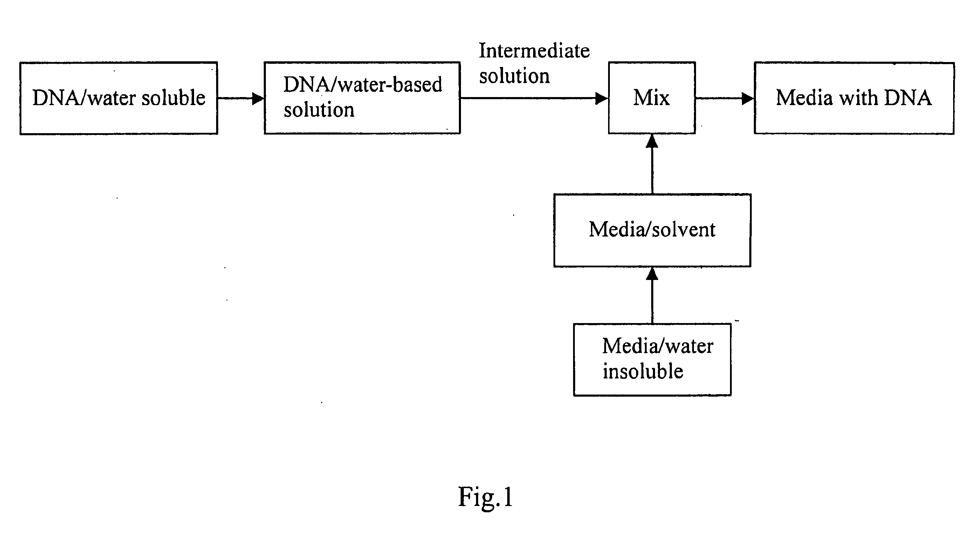

Fig. 1 shows a flowchart regarding the process of mixing ribonucleic acid in water insoluble media of the invention.

The following is a detailed description of the best presently known modes of carrying out the inventions. of the inventions.

Since nucleic acid is highly water-soluble material, it is easily dissolved in water-soluble solutions, such as PBS or TE. On the other hand, it is not easy to mix ribonucleic acid in water insoluble media. Thus, the invention is to provide with a method for resolving ribonucleic acid in water insoluble media.

As shown in Fig. 1, a method for mixing ribonucleic acid in water insoluble media in the invention comprises the procedures as follows: first, respectively dissolving nucleic acid in water-soluble solutions and water insoluble media in solvents, and then mix nucleic acid solution in the media by utilizing a particular intermediate solution, thus media having nucleic acid can be obtained.

The foregoing media can be polypropylene (PP), polycarbonate, or preferably polystyrene (PS).

The foregoing intermediate is an organic solvent, such ,as ethanol, acetone, chloroform or the mixtures thereof.

Another objective of the invention is to provide a method for producing product anti-counterfeiting labels, with characteristics being that: mixing known sequences nucleic acid in water insoluble media by using the method described above, and then coating the media on the surface of objects or enabling the media to infiltrate such objects as labels. The foregoing media are utilized for mixing and protecting nucleic acid, and for adhering or mixing in other objects.

The foregoing objects can be liquids or solid objects, wherein liquid objects, such as inks, paints, pigments, cosmetics, seals or glues, can be mixed with media having ribonucleic acid, thus enabling such liquids to be anti-counterfeiting labels. For example, after mixing nucleic acid with polypropylene media through a particular intermediate solution, the powder or minuscule particles of polypropylene media are to be mixed with inks, thus anti-counterfeiting inks having nucleic acid are to be obtained.

In addition, solid objects, such as antiques, jewelries, credit cards, magnetic strip cards, souvenirs, can be attached thereon with media having nucleic acid, thus enabling such solid objects to provide anti-counterfeiting functions.

Apart from the foregoing applications, media having nucleic acid can directly be utilized as product material, thus enabling the products themselves having the function of anti-counterfeiting. For example, to mix nucleic acid with polystyrene or polypropylene media through a particular intermediate solution shall make such media become material having the anti-counterfeiting function, thus such material can be widely utilized for making all kinds of anti-counterfeiting products or labels.

The foregoing nucleic acid is the general term for deoxyribonucleic acid (DNA) or ribonucleic acid (RNA), and can be chosen from animals, plants, bacteria, viruses, fungi, or synthetic vectors or fragments.

The identification method by utilizing nucleic acid as product anti-counterfeiting labels mentioned above is to detach a small portion of the media from objects, and then recycle nucleic acid having known sequences in such media by utilizing solvents to extract therefrom, and the composition of nucleic acid was designed to have specific length and sequence which can only be verified with certain PCR primers. Through this procedure, if the examined object carries the original nucleic acid, the PCR procedure will amplify extracted nucleic acid to several million times with the same size and sequence of the original nucleic acid. In contrast, if the media detached from the examined object does not have the original nucleic acid, there will be no amplified nucleic acid product. Therefore, by comparing the sizes and amount of PCR products, the authenticity of labeled objects can be verified.

The invention provides a method for mixing nucleic acid in water insoluble media, with the detailed manufacturing method being further elaborated through the preferred embodiment as follows.

The preferred embodiment describes the method for mixing the water-soluble DNA into PS.
- Material:: Polystyrene (PS) is utilized as the medium, the organic solvent is chloroform, and the intermediate solution is 95 % of ethanol, acetone and chloroform.
- Method:: At first, dissolve 5 µ g of the prepared DNA into 100 µl of water, and dissolve 5g of PS into chloroform, afterwards add respectively 10 µl of intermediate solution of 95% ethanol and acetone into the foregoing DNA water solution, and eventually, fully mix, through vigorous vortex, the foregoing DNA solution having the intermediate added therein with the organic solution of chloroform having PS dissolved therein. Through such intermediate process, the DNA water solution and the water insoluble medium of PS chloroform are to be thoroughly mixed, thus the medium of PS solution containing the DNA is obtained.

Although the present invention has been described in considerable detail with reference to certain preferred embodiments thereof, those skilled in the art can easily understand that all kinds of alterations and changes can be made thereto.

## Claims

1. A method for mixing nucleic acid into a water insoluble medium,
wherein said medium is polypropylene (PP), polycarbonate (PC) or polystyrene, comprising processes as follows:
dissolving nucleic acid into water-soluble solution;
dissolving said water insoluble medium of polypropylene, polycarbonate or polystyrene into a solvent;
by addition of a particular intermediate solution, which is ethanol, acetone, chloroform or mixtures thereof, to said solution containing nucleic acid, and mixing of said solution containing nucleic acid, with said solvent containing said water insoluble media;
a medium containing nucleic acid being obtained.

2. A method for mixing nucleic acid into said water insoluble medium as in claim 1, wherein said medium is polystyrene.

3. A method for mixing nucleic acid into a water insoluble medium as in claim 1, wherein said medium containing nucleic acid can directly be utilized as product material, thus making said product have an anti-counterfeiting function.

4. A method for mixing nucleic acid into a water insoluble medium as in claim 1, wherein said nucleic acid is the general term for deoxyribonucleic acid (DNA) or ribonucleic acid (RNA).

5. A method for mixing nucleic acid into a water insoluble medium as in claim 1, wherein said nucleic acid is chosen from animals, plants, bacteria, viruses, fungi or synthetic vectors or fragments, thereof.

## Patentansprüche

1. Verfahren zum Einmischen von Nucleinsäure in ein wasserunlösliches Medium,
wobei das Medium Polypropylen (PP), Polycarbonat (PC) oder Polystyrol ist, das die folgenden Prozesse umfaßt:
Lösen von Nucleinsäure in einer wasserlöslichen Lösung;
Lösen des wasserunlöslichen Mediums aus Polypropylen, Polycarbonat oder Polystyrol in einem Lösungsmittel;
wobei durch die Zugabe einer bestimmten intermediären Lösung, die Ethanol, Aceton, Chloroform oder Gemische davon ist, zu der Nucleinsäure enthaltenden Lösung und Mischen der Nucleinsäure enthaltenden Lösung mit dem Lösungsmittel, das das wasserunlösliche Medium enthält,
ein Medium erhalten wird, das Nucleinsäure enthält.

2. Verfahren zum Einmischen von Nucleinsäure in ein wasserunlösliches Medium nach Anspruch 1, wobei das Medium Polystyrol ist.

3. Verfahren zum Einmischen von Nucleinsäure in ein wasserunlösliches Medium nach Anspruch 1, wobei das Nucleinsäure enthaltende Medium direkt als Produktmaterial verwendet werden kann, womit das Produkt folglich eine vor Nachahmung schützende Funktion erhält.

4. Verfahren zum Einmischen von Nucleinsäure in ein wasserunlösliches Medium nach Anspruch 1, wobei Nucleinsäure der allgemeine Begriff für Desoxyribonucleinsäure (DNA) oder Ribonucleinsäure (RNA) ist.

5. Verfahren zum Einmischen von Nucleinsäure in ein wasserunlösliches Medium nach Anspruch 1, wobei die Nucleinsäure aus Tieren, Pflanzen, Bakterien, Viren, Pilzen oder synthetischen Vektoren oder Fragmenten davon ausgewählt ist.

## Revendications

1. Procédé pour mélanger un acide nucléique dans un milieu insoluble dans l'eau, dans lequel ledit milieu est le polypropylène (PP), un polycarbonate (PC) ou le polystyrène, comprenant les opérations suivantes :
la dissolution de l'acide nucléique dans une solution soluble dans l'eau ;
la dissolution du dit milieu insoluble dans l'eau de polypropylène, polycarbonate ou polystyrène, dans un solvant ;
par addition d'une solution intermédiaire particulière, qui est de l'éthanol, de l'acétone, du chloroforme ou des mélanges de ceux-ci, à ladite solution contenant l'acide nucléique, et le mélange de ladite solution contenant l'acide nucléique avec ledit solvant contenant lesdits milieux insolubles dans l'eau ;
un milieu contenant un acide nucléique étant obtenu.

2. Procédé pour mélanger un acide nucléique dans ledit milieu insoluble dans l'eau, selon la revendication 1, dans lequel ledit milieu est le polystyrène.

3. Procédé pour mélanger un acide nucléique dans un milieu insoluble dans l'eau, selon la revendication 1, dans lequel ledit milieu contenant l'acide nucléique peut être directement employé comme produit, ce qui confère ainsi au dit produit une fonction anti-contrefaçon.

4. Procédé pour mélanger un acide nucléique dans un milieu insoluble dans l'eau, selon la revendication 1, dans lequel ledit acide nucléique est le terme général pour l'acide désoxyribonucléique (ADN) ou l'acide ribonucléique (ARN).

5. Procédé pour mélanger un acide nucléique dans un milieu insoluble dans l'eau, comme dans la revendication 1, dans lequel ledit acide nucléique est choisi parmi les animaux, les végétaux, les bactéries, virus, les champignons ou les vecteurs synthétiques ou les fragments de ceux-ci.
